# EUROPEAN PATENT APPLICATION

(11) **EP 3 915 609 A1**
(43) Date of publication of application: **01.12.2021**
(21) Application number: 21169697.6
(22) Date of filing: 21.04.2021
(51) Int. Cl.: A61M 1/36, B01D 29/11, B01D 29/58, B01D 69/02

(54) **MEDICAL DEVICE FOR THE TREATMENT OF BODILY SUBSTANCES**

(30) Priority: 26.05.2020 IT 202000012355
(71) Applicant: Tecnoline S.p.A., 41033 Concordia Sulla Secchia, MO (IT)
(72) Inventor: PROVASI, Stefano, 41033 CONCORDIA SULLA SECCHIA MO (IT)
(74) Representative: Modiano, Micaela Nadia

(57) **Abstract**

A medical device for the treatment of bodily substances, which comprises at least one pouch (2) which has a chamber (3) inside it for the treatment of bodily substances and which is provided with at least one inlet (4) and at least one outlet (5), respectively for the introduction into the chamber (3) and the evacuation from the latter of the bodily substance to be treated.

The device comprises at least one filter (6), which is arranged in the chamber (3) and which is configured for the filtration of the entirety of the substances in transit from the inlet (4) to the outlet (5); the filter (6) is substantially shaped like a pocket defining an access mouth that is open toward the inlet (4) and the entire rim of such mouth is coupled to the pouch (2), which in turn is made of deformable material.

## Description

The present invention relates to a medical device for the treatment of bodily substances.

In the medical sector, the need often arises to subject the blood of a patient to a filtration treatment, in order to remove impurities of various kinds and so prepare it for reintroduction back into the body of the patient.

For example, this requirement arises in relation to surgical procedures of various types, during which the patient can lose great amounts of blood which must be replenished in some way. In such an eventuality, the reinfusion of the patient's own blood, adequately filtered, is preferred to a transfusion from a different person in that, as is known, the introduction of cells and fluids that in any case are foreign to the organism entails non-negligible risks of rejection and other complications.

To this end, filtering devices are therefore known which comprise a pouch inside which one or two filtering membranes are arranged, which are shaped like simple flattened sheets that extend for the full extension of the pouch (being welded to the rims thereof), thus subdividing the chamber inside the pouch into respective compartments. The blood is introduced into the chamber in a first opening made in the pouch and is made to exit at the opposite end, in a second opening of the pouch, not before having forcibly passed through the membranes.

Such implementation solution is however not devoid of drawbacks.

The membranes are simple sheets arranged mutually parallel (and parallel to the side walls of the pouch): in order to prevent them from sticking together, or to the pouch, thus compromising the filtration efficacy, it is necessary to stiffen the pouch and/or the membranes, so that these components are kept mutually spaced apart in any case.

This however brings numerous drawbacks: first of all in fact, the structure of the device is complicated, resulting in an unwanted increase in the cost.

Furthermore, the need to keep the membranes and/or the walls of the pouch mutually spaced apart appreciably increases the encumbrance of the device, which however cannot be reduced owing to the measurements adopted to stiffen it. This is found to be exceedingly unwelcome when, conversely, one wishes to have the possibility to fold it up in order to transport it or store it in a more convenient manner.

It should lastly be noted that devices of the type described above are also adopted for treating other bodily substances or fluids: in these contexts too (for example the filtration and cleaning of body fat, in cosmetic surgery), it is possible to encounter the drawbacks indicated above.

The aim of the present invention is to solve the above mentioned problems, by providing a medical device that makes it possible to treat, and in particular filter, blood or other bodily substances in an optimal manner.

Within this aim, an object of the invention is to provide a medical device that enables a complete filtration of the blood or of other bodily substances, while at the same time adopting a simple structure.

Another object of the invention is to provide a medical device that can be easily folded up when it is not in use.

Another object of the invention is to provide a medical device that ensures a high reliability of operation and which is versatile, because it can be easily used in different contexts and for the treatment of various substances.

Another object of the invention is to provide a medical device that adopts an alternative technical and structural architecture to those of conventional medical devices.

Another object of the invention is to provide a medical device that can be easily implemented using elements and materials that are readily available on the market.

Another object of the invention is to provide a medical device that is of low cost and safely applied.

This aim and these and other objects which will become better apparent hereinafter are achieved by a medical device for the treatment of bodily substances, which comprises at least one pouch with a chamber inside it for the treatment of bodily substances and provided with at least one inlet and at least one outlet, respectively for the introduction into said chamber and the evacuation from said chamber of the bodily substance to be treated, characterized in that it comprises at least one filter, which is arranged in said chamber and is configured for the filtration of the entirety of the substances in transit from said inlet to said outlet, said at least one filter being substantially shaped like a pocket defining an access mouth that is open toward said inlet, the entire rim of said mouth being coupled to said pouch, made of deformable material.

Further characteristics and advantages of the invention will become better apparent from the description of a preferred, but not exclusive, embodiment of the medical device according to the invention, which is illustrated by way of non-limiting example in the accompanying drawings wherein:
Figure 1 is a perspective view of the medical device according to the invention;
Figure 2 is a perspective view of a portion of the device of Figure 1, cross-sectioned along a plane perpendicular to the pouch.

With particular reference to the figures, the reference numeral 1 generally designates a medical device for the treatment of bodily substances. In particular, in an application of significant practical interest the device 1 can be used for an efficacious filtration of the blood, so as to remove impurities of various kinds therefrom and be able to subsequently reintroduce it into the body of a patient (from which it may have been collected previously, for example during a surgical operation).

In a second possible application of the invention, the device 1 is instead designed for the filtration and the treatment of body fat, in cosmetic surgery, where the need sometimes arises to collect masses of fat from a subject in order to filter it, purify it and optionally remove stem cells, botulin or the like (in this case too subsequently proceeding to reintroduce the treated fat into the body of the patient).

It should be noted however that the scope of protection claimed herein should be understood as being extended also to the treatment (filtration and the like) of various different substances, according to the specific requirements.

The device 1 comprises at least one pouch 2, which has a chamber 3 inside it for the treatment of bodily substances; the pouch 2 is further provided with at least one inlet 4 and at least one outlet 5, respectively for introducing the bodily substance to be treated into the chamber 3 and for evacuating it from that chamber 3.

In any case the pouch 2 will be provided with any desired number of inlets 4 and outlets 5 and the former will be located at the opposite end to the latter, with respect to the pouch 2, so that it is possible to arrange them (when the pouch 2 is hung or howsoever in use) in such a way that the inlets 4 are in an upward region and the outlets 5 in a downward region. In this manner, the bodily substances are pushed toward the outlets 5 simply by gravity, after they have been introduced in the inlets 4.

It should further be emphasized that each inlet 4 or outlet 5 can be provided with connectors, valves and/or any other similar component that the person skilled in the art may deem it appropriate to implement, in order to facilitate the experience of use of the device 1 and/or to increase its safety and its reliability, in order to facilitate connection to other instruments, etc.

According to the invention, the medical device 1 comprises at least one filter 6, which is arranged in the chamber 3 and which is configured to filter all the substances in transit from the inlet 4 to the outlet 5 (i.e., it is arranged so as to intercept all of the substances in transit).

The filter 6 is substantially shaped like a pocket that defines an access mouth that is open toward the inlet 4 (or each inlet 4); the entire rim of such mouth (which extends transversely for the entire transverse cross-section of the pouch 2) is coupled to the pouch 2, which in turn is made of deformable material. The coupling to the pouch 2 is preferably but not exclusively obtained by heat-sealing.

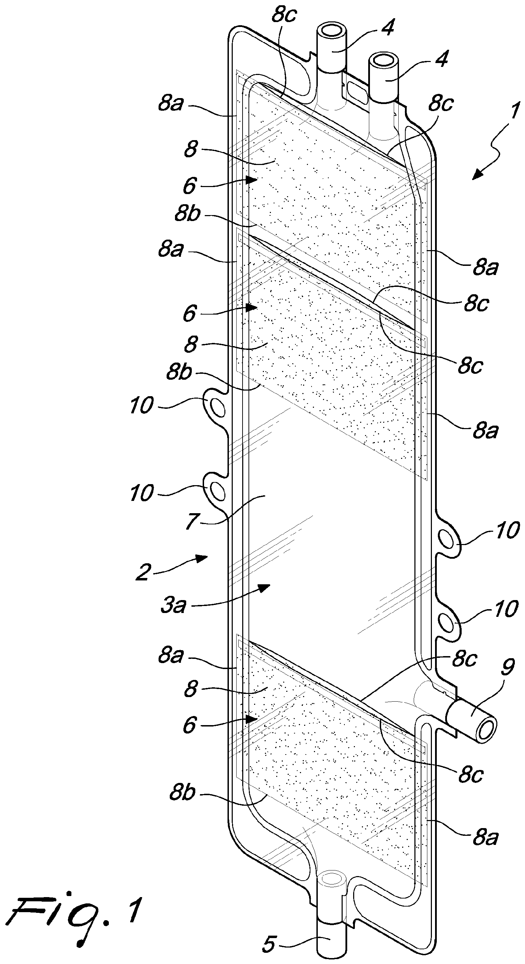

In particular, in an embodiment of significant practical interest, also illustrated in the accompanying figures for the purposes of non-limiting example of application of the invention, the medical device 1 comprises a plurality of filters 6, which are arranged in series inside the chamber 3 between the inlet 4 and the outlet 5. By virtue of the arrangement in series, the bodily substances in transit in the chamber 3 are forced to pass through each filter 6 before they can exit at the outlet 5. When the pouch 2 is kept with the inlet 4 in an upper region and the outlet 5 in a lower region (as in the normally envisaged conditions of use), the filters 6 are therefore aligned vertically, as shown also in Figure 1.

More specifically, the filters 6 have a porosity that decreases progressively from the inlet 4 to the outlet 5. It should be noted that the term "porosity" here means the average size of the holes that each filter 6 has (through which the bodily substances are forced to pass during the filtering).

In this manner, the first filter 6 that the bodily substances introduced into the chamber 3 encounter acts to retain only the larger particles and impurities, while the filters 6 downstream retain and remove progressively smaller ones.

In the preferred embodiment, illustrated in the accompanying figures for the purposes of non-limiting example of the invention, the pouch 2 is constituted substantially by two mutually opposite (flattened) flexible flaps 7, which are mutually coupled along the respective perimeters (where the inlet 4 and the outlet 5 are located). The flaps 7 preferably but not exclusively have a rectangular outline, as indeed in the accompanying figures.

With further reference to the preferred solution, illustrated in the accompanying figures, the filter 6 (each filter 6) is substantially constituted by two rectangular sheets 8 (optionally square and/or partially rounded at the corners) which are mutually coupled at three common sides 8a, 8b and which define, with respective fourth sides 8c, the above mentioned rim of the access mouth.

Two common sides 8a of each sheet 8, which are contiguous to the fourth side 8c, are integrally clamped between respective sides of the flaps 7 (and therefore of the pouch 2), which in turn are mutually heat-sealed.

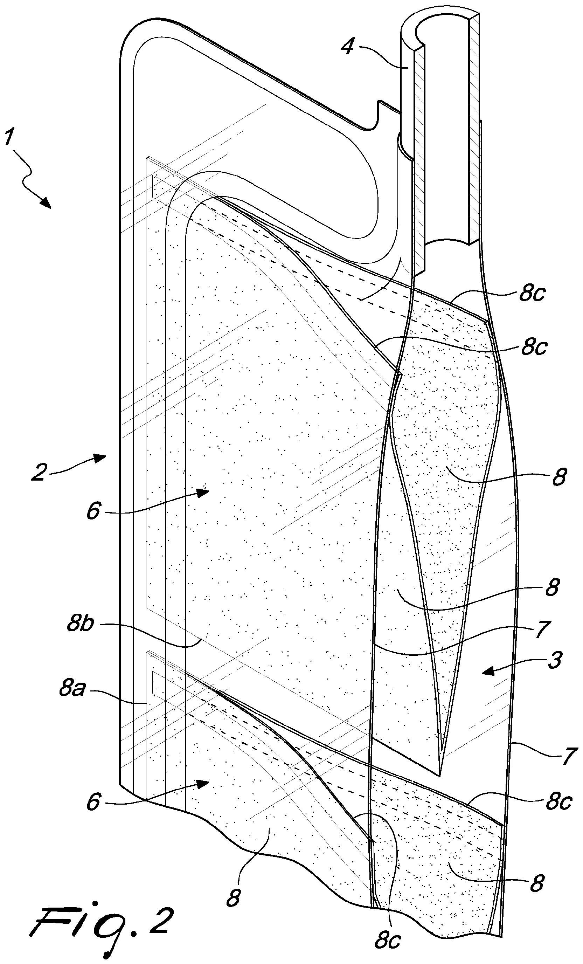

At the same time, as Figure 2 clearly shows, the other common sides 8b (opposite to the fourth sides 8c, which are obviously kept mutually independent), are integrally coupled (heat-sealed) to each other, but are detached from the flaps 7.

It is noted in any case that the shape of each filter 6 can also be different, with respect to the shape described above, while remaining within the scope of protection claimed herein.

Usefully, the pouch 2 has at least one auxiliary opening 9, which is connected to a compartment 3a of the chamber 3 which is interposed between two filters 6, which are consecutive but are kept mutually spaced apart. Note that for the purposes of the invention (which will be better illustrated in the following pages) the two filters 6 can be kept for example at a distance comprised between 5 cm and 15 cm, and for example at 10 cm or 11 cm. However, the possibility is not ruled out of a different dimensioning of the compartment 3a.

It should be noted that there is the possibility of arranging each filter 6 immediately downstream of the previous one (a few millimeters from the latter), i.e. in such a way that the access mouth of each downstream filter 6 is facing and proximate to the respective upstream filter 6.

Alternatively, at least two consecutive filters 6 can indeed be kept mutually spaced apart, so as to define a compartment 3a of the chamber 3 in which the bodily substances can transit or be temporarily accommodated, without being affected by the action of the filters 6. In such case therefore, there is in fact possibility of providing the pouch 2 with an auxiliary opening 9 that provides direct access to the compartment 3a.

Conveniently, the device 1 comprises at least one lug 10, which protrudes externally from the pouch 2 (from the edges of the flaps 7) at the compartment 3a and which is configured for coupling with a clamp (or a clip, or other similar securing body), which can be fastened around the pouch 2 for the isolation of the compartment 3a from the inlet 4.

This clamp can be supplied together with the device 1 or supplied separately, and can in any case also be chosen to be of a type that is per se known.

While stating that the pouch 2 can be made of any material according to the specific requirements, in an embodiment of significant practical interest it is made of an polymeric material, preferably chosen between polyvinyl chloride and polyurethane.

Each filter 6 can also be made of any material (preferably deformable), while remaining within the scope of protection claimed herein, but in a practical solution of particular interest, which in any case is cited here purely for the purposes of example, it is made of polyester.

The operation of the medical device according to the invention is the following.

Through the inlet 4, it is possible to introduce blood or other substances to be treated, and in particular to be filtered, into the chamber 3.

By arranging the pouch 2 so that the inlet 4 is in an upward region and the outlet 5 in a downward region, for example by hanging it conveniently from a respective support, the blood introduced moves by gravity toward the outlet 5 and, in doing so, passes through the filters 6 in sequence, which take care of removing the unwanted impurities and particles of various nature contained in the blood (as a function obviously of the porosity of each filter 6 that has been chosen to be accommodated in the chamber 3). The possibility is not ruled out however of having means that are actively responsible for the advancement of the substance to be treated in the chamber 3.

The blood (or other substance) that exits from the outlet 5 is therefore effectively purified and can be re-infused in the same subject from which it was taken, or indeed in another subject, according to the specific requirements.

In other possible applications, the auxiliary opening 9 can be used to make the treated substance exit before it passes through the filters 6 downstream, or to introduce an additional fluid (or gas, or the like) into the chamber 3, with which to carry out an additional treatment on the bodily substance, before the substance exits from the chamber 3 (from the outlet 5 or from the opening 9).

In particular, in an application of significant practical interest, body fat is introduced into the chamber 3, through the inlet 4, leaving the entire mass involved pass through the filters 6 until it reaches the compartment 3a (thus being purified and, at the same time, optionally also obtaining a reduction in the molecules that make it up).

When the fat has reached the compartment 3a, the latter is then isolated from the inlet 4 (using the clamp associated with the lugs 10) and a washing liquid is injected into the compartment 3a (through the opening 9), with which to clean the entire mass of fat. Preferably this step is carried out while keeping the pouch 2 horizontal or inverted, in order to prevent the fat from passing through the downstream filters 6 and exiting from the outlet 5 before the washing is complete.

At the end of the washing, the fat can be made to exit directly from the opening 9 or it can be left to be filtered by the downstream filters 6, in order to then be made to flow through the outlet 5.

It should be noted that the two treatments described above are cited purely for the purposes of example: in fact the use of the invention is also adapted for different bodily substances and/or for different purposes (and with different methods).

In any case, it can be seen that upon introducing bodily substances into the pouch 2, the two flaps 7 (or in any case the walls of the pouch 2) tend to become mutually spaced apart, and with them therefore the access mouth also tends to open (it being coupled to the flaps 7 or in any case to the pouch 2 itself). In this manner, the pocket outlined by the filter 6 opens and receives the substance to be treated, while at the same time guarding against the risk that the various surfaces of the filter 6 might adhere to each other, thus compromising the filtration efficacy. Likewise, the filter 6 also does not adhere to the walls of the pouch 2, being coupled to it only along the rim of its access mouth (and preferably along the two common sides 8a).

When the device 1 has two or more filters 6, they are arranged in a cascade and evidently it is not possible for them to adhere to each other, again ensuring maximum efficacy and an optimal operation.

Conversely, when the device 1 is not in use, its encumbrance can be easily reduced by folding and rolling the pouch 2 (which to this end is made of deformable material); if it is used again, it is sufficient to restore the extended configuration and then proceed with the introduction of a new substance, and still be able to count on the full efficacy of filtration, in that evidently the filters 6 do not adhere to each other or to the walls (the flaps 7) of the pouch 2, for the same reasons just explained.

The medical device 1 therefore makes it possible to treat, and in particular to filter, blood or other bodily substances in an optimal manner, thus ensuring a complete filtration of the blood or of other bodily substances, while at the same time adopting a simple structure that does not require the stiffening of any parts thereof or the use of further components that keep the filters 6 forcibly spaced apart (or each filter 6 spaced apart from the pouch 2), as happens in conventional solutions.

The extreme simplicity of the invention, which can be obtained with few elements which are readily available on the market, makes it particularly reliable and susceptible of industrialization at low cost.

It has likewise been noted that the medical device is absolutely versatile, because it can be easily used in different contexts and for the treatment of various substances.

The invention, thus conceived, is susceptible of numerous modifications and variations, all of which are within the scope of the appended claims. Moreover, all the details may be substituted by other, technically equivalent elements.

In the embodiments illustrated, individual characteristics shown in relation to specific examples may in reality be substituted with other, different characteristics, existing in other embodiments.

In practice, the materials employed, as well as the dimensions, may be any according to requirements and to the state of the art.

The disclosures in Italian Patent Application No. 102020000012355 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A medical device for the treatment of bodily substances, which comprises at least one pouch (2) with a chamber (3) inside it for the treatment of bodily substances and provided with at least one inlet (4) and at least one outlet (5), respectively for the introduction into said chamber (3) and the evacuation from said chamber (3) of the bodily substance to be treated, **characterized in that** it comprises at least one filter (6), which is arranged in said chamber (3) and is configured for the filtration of the entirety of the substances in transit from said inlet (4) to said outlet (5), said at least one filter (6) being substantially shaped like a pocket defining an access mouth that is open toward said inlet (4), the entire rim of said mouth being coupled to said pouch (2), made of deformable material.

2. The medical device according to claim 1, **characterized in that** it comprises a plurality of said filters (6), which are arranged in series inside said chamber (3) between said inlet (4) and said outlet (5).

3. The medical device according to claim 2, **characterized in that** said filters (6) have a porosity that decreases progressively from said inlet (4) to said outlet (5).

4. The medical device according to one or more of the preceding claims, **characterized in that** said pouch (2) is substantially constituted by two mutually opposite flexible flaps (7), which are mutually coupled along the respective perimeters and preferably have a rectangular outline.

5. The medical device according to claim 4, **characterized in that** said at least one filter (6) is substantially constituted by two rectangular sheets (8) which are mutually coupled at three common sides (8a, 8b) and which define, with respective fourth sides (8c), said rim of said access mouth, two of said common sides (8a) of each one of said sheets (8), which are contiguous to said fourth side (8c), being integrally clamped between respective sides of said flaps (7), which are mutually heat-sealed.

6. The medical device according to one or more of the preceding claims, **characterized in that** said pouch (2) has at least one auxiliary opening (9), which is connected to a compartment (3a) of said chamber (3) which is interposed between two of said filters (6), which are consecutive but are kept mutually spaced apart.

7. The medical device according to claim 6, **characterized in that** it comprises at least one lug (10) which protrudes externally from said pouch (2) at said compartment (3a) and is configured for coupling with a clamp, which can be fastened around said pouch (2) for the isolation of said compartment (3a) from said inlet (4).

8. The medical device according to one or more of the preceding claims, **characterized in that** said pouch (2) is made of a polymeric material, preferably chosen between polyvinyl chloride and polyurethane.

9. The medical device according to one or more of the preceding claims, **characterized in that** said at least one filter (6) is made of polyester.
